Europäisches Patentamt

European Patent Office (11) Numéro de publication : **0 073 772**

Office européen des brevets **B1**

(19)

(12) **FASCICULE DE BREVET EUROPÉEN**

(45) Date de publication du fascicule du brevet :
30.12.86

(51) Int. Cl.⁴ : **A 61 F 5/04**, A 61 F 5/01,
B 32 B 15/08

(21) Numéro de dépôt : 82900549.5

(22) Date de dépôt : 19.02.82

(86) Numéro de dépôt international :
**PCT/FR 82/00030**

(87) Numéro de publication internationale :
**WO/8203005 (16.09.82 Gazette 82/22)**

(54) **Matériau pour la fabrication de gouttières et minerves.**

(30) Priorité : 10.03.81 FR 8104715

(43) Date de publication de la demande :
**16.03.83 Bulletin 83/11**

(45) Mention de la délivrance du brevet :
**30.12.86 Bulletin 86/52**

(84) Etats contractants désignés :
**BE**

(56) Documents cités :
**EP-A- 0 005 615**
**FR-A- 1 515 909**
**FR-A- 2 079 329**
**FR-A- 2 248 152**
**US-A- 3 055 358**

(73) Titulaire : **ETABLISSEMENTS THUASNE & CIE**
**27, rue de la Jomayère**
**F-42031 St. Etienne Cedex (FR)**

**THUASNE-PARIS**
**73, rue du Faubourg St. Martin**
**F-75010 Paris (FR)**

(72) Inventeur : **PICOLET, Jean-Pierre**
**24, rue des Vergers**
**F-42270 St. Priest-en-Jarez (FR)**
Inventeur : **RUDLOFF, Jean-Loup**
**28, rue de Stalingrad**
**F-92000 Nanterre (FR)**
Inventeur : **COURTET, François**
**La Cote**
**F-42330 St. Bonnet-les-Oules (FR)**

(74) Mandataire : **Coutel, Jean-Claude**
**Cabinet AYMARD & COUTEL 20, rue Vignon**
**F-75009 Paris (FR)**

EP 0 073 772 B1

Jouve, 18, rue St-Denis, 75001 Paris, France

## Description

L'invention concerne un matériau pour la confection d'articles de maintien de la tête ou de membres de l'homme ou d'animaux.

On connaît déjà, depuis longtemps, de nombreux articles de ce genre, mais ils présentent un certain nombre d'inconvénients. Ainsi, pour l'immobilisation d'une jambe fracturée, on connaît le store Jeanbrau qui comporte des lattes de bois parallèles reliées entre elles comme celles d'un store et qui permet de confectionner une gouttière de secours d'urgence, mais il présente l'inconvénient d'être encombrant et nécessite la mise en place d'un rembourrage de protection du membre blessé. L'attelle de Kramer, formée d'un grillage métallique, n'est déformable que dans un seul plan et nécessite aussi un rembourrage de protection dont la mise en place est assez longue ; de plus, elle est opaque aux rayons X. L'attelle pneumatique entoure le membre et assure son immobilisation par le fait qu'elle est gonflable, mais elle ne permet pas de respecter une déformation et elle est relativement fragile. La gouttière métallique réalisée en grillage nécessite un rembourrage de protection, elle ne permet pas de respecter une déformation supérieure à la largeur de la gouttière et elle est opaque aux rayons X.

On connaît, par le document FR-A-2 079 329, une attelle stratifiée pour membres malades ou fracturés, cette attelle comportant une âme métallique, constituée par exemple par des bandes de tôle, noyée dans un rembourrage constitué par une matière alvéolaire élastique et revêtu en surface d'une couche étanche à l'eau. Dans cette attelle, le rembourrage évite le contact direct, ou quasi direct à travers la couche étanche, de l'âme métallique avec le membre à maintenir, mais il n'existe, d'une part, aucune protection efficace contre les chocs extérieurs et, d'autre part, aucun moyen pour amortir et répartir les déformations des bandes de tôle, ces déformations constituant une cause de rupture de celles-ci si elles sont concentrées sur une faible zone.

On connaît encore, par le document EP-A-000 615, un dispositif protecteur stratifié comportant une couche intérieure cellulaire souple et une couche extérieure cellulaire qui peut être déformée par flexion de manière plastique pour déterminer la rigidité de l'ensemble à l'état déformé. La couche extérieure peut porter un revêtement flexible dur de compression et de rigidité. Dans un tel dispositif, ou bien le revêtement dur est présent, auquel cas il exerce une force de compression excessive du fait qu'il est inextensible et à l'extérieur du stratifié, ou bien ce revêtement dur n'existe pas et le produit ne présente pas de protection mécanique contre le milieu extérieur et n'est pas suffisamment raide.

Le but de l'invention est de proposer un matériau qui ne présente pas les inconvénients précités rappelés plus haut.

A cet effet, le matériau selon l'invention, pour la confection d'articles de maintien de la tête ou des membres de l'homme ou des animaux, est constitué d'un panneau stratifié formé par l'assemblage d'une couche extérieure de matière plastique sous forme de mousse, d'une âme intermédiaire en métal déformable et non élastique, et d'une couche intérieure de matière plastique sous forme de mousse souple, et il est caractérisé en ce qu'il comporte une feuille intérieure extrême souple et lisse de protection en ce que l'âme intermédiaire est formée d'une feuille de métal et en ce que la couche extérieure est en mousse dure. La couche de matière plastique sous forme de mousse dure peut être recouverte, soit d'une feuille extérieure souple et lisse auquel cas on maintiendra les articles confectionnés à partir d'un tel matériau au moyen de bandes ou sangles munies de moyens de fixation (boucles, « Velcro », etc...), soit d'un matériau à bouclettes souples, tel que tricot ou tissu gratté et, dans ce cas, on pourra alors utiliser des sangles munies d'éléments propres à s'accrocher directement dans ledit matériau à bouclettes.

Le plus souvent, le panneau et surtout la feuille métallique qu'il comporte, seront découpés suivant des formes prédéterminées correspondant aux conformations des divers articles à confectionner, gouttières diverses ou minerves, par exemple. De toute façon, ainsi qu'on le comprendra mieux plus loin, au cours de l'exposé qui va suivre, on conçoit, dès maintenant, que le matériau en question permet de confectionner des articles faciles à mettre en place sans réglages précis grâce à la plasticité du produit qui le rend modelable et, par conséquent, d'une grande souplesse d'utilisation, notamment en respectant des déformations importantes d'un membre, possédant un rembourrage intégré, solides, légers, utilisables en montagne car ils ne craignent pas le gel, fiables pendant les déplacements, et transparents aux rayons X.

L'invention sera mieux comprise à la lecture de la description qui va suivre et à l'examen des dessins annexés qui montrent, à titre d'exemples, la structure d'un panneau en matériau suivant l'invention et quelques articles de maintien et de protection confectionnés à partir d'un tel matériau.

Sur ces dessins :

Figure 1 représente en coupe faite suivant la ligne I-I de la fig. 2, un panneau pour la confection d'une gouttière suivant l'invention, pour un membre inférieur fracturé ;

Figure 2 est, à plus grande échelle, une coupe faite suivant la ligne II-II de la fig. 1 ;

Figures 3 et 4 sont, respectivement, des vues de profil et en plan d'une gouttière confectionnée à partir du panneau des fig. 1 et 2, modelée et fixée sur un membre inférieur fracturé ;

Figure 5 représente de face un panneau (dont la couche de matière plastique sous forme de mousse souple et la feuille intérieure de protection sont supposées transparentes) pour la confection d'une gouttière de maintien de la zone avant-bras, coude, partie inférieure du bras ;

Figure 6 montre, en perspective, une gouttière confectionnée à partir du panneau de la fig. 5, modelée et fixée sur un bras ;

Figure 7 représente de face un panneau (également dans lequel la couche de matière plastique sous forme de mousse souple et la feuille intérieure de protection sont supposées transparentes) pour la confection d'une gouttière pour fracture de poignet ;

Figures 8 et 9 sont, respectivement, des vues de face et en plan d'une gouttière confectionnée à partir du panneau de la fig. 7 modelée et fixée dans la zone d'un poignet ;

Figures 10 et 11 représentent, respectivement, deux panneaux (toujours avec la même partie supposée transparente) pour la confection d'une minerve ;

Figure 12 montre en perspective, la manière de conformer les deux panneaux des fig. 10 et 11 pour la préparation d'une minerve en deux parties ;

Figure 13 illustre, en perspective, la manière de fixer la minerve sur le patient ; et

Figure 14 montre, en coupe transversale, un autre mode de réalisation de gouttière.

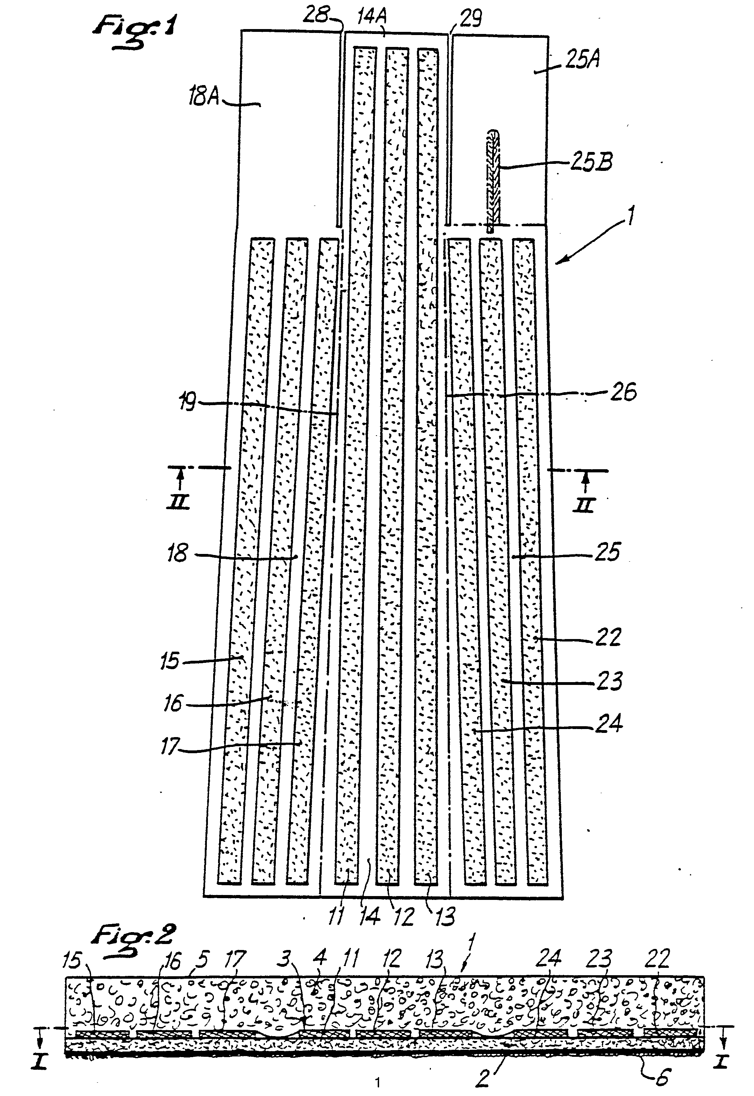

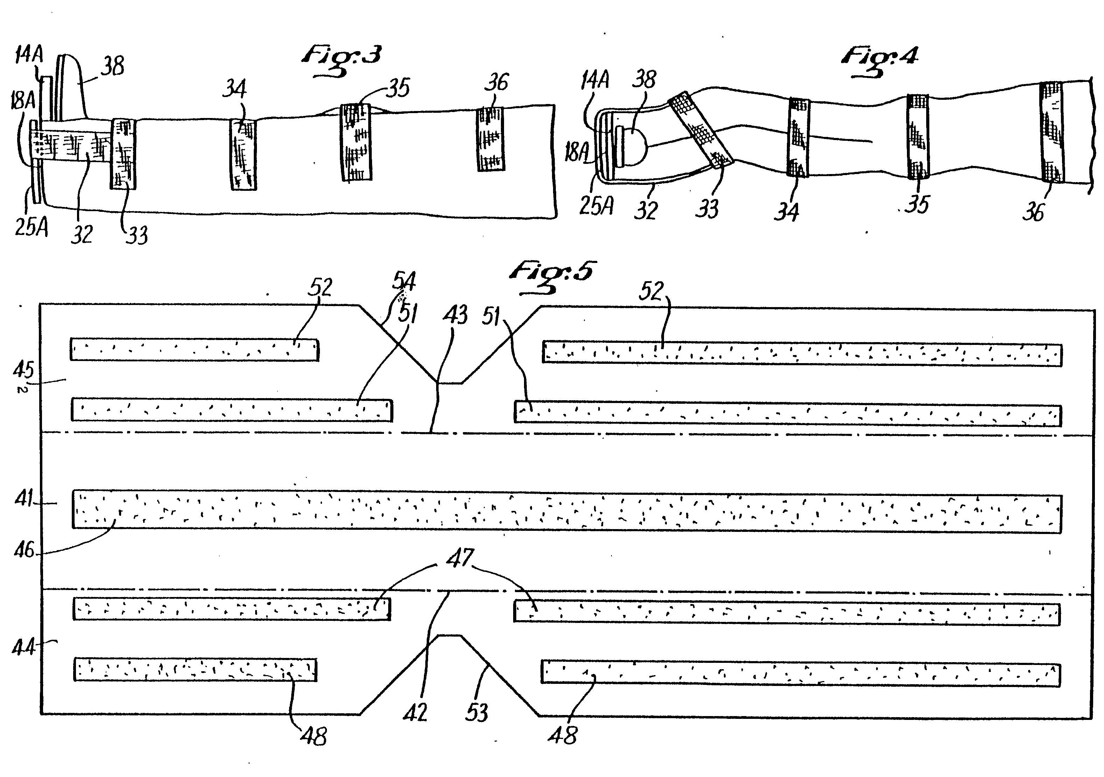

Le panneau 1 représenté sur les fig. 1 et 2 est destiné à la confection d'une gouttière à modeler et fixer sur un membre inférieur fracturé ; il est en un matériau composite stratifié formé par l'assemblage d'une couche de matière plastique sous forme de mousse dure 2, d'une feuille de métal 3, de préférence non élastique mais facilement déformable, d'une couche de matière plastique sous forme de mousse souple 4, et d'une feuille intérieure souple et lisse de protection 5 ; de plus, la couche de matière plastique sous forme de mousse dure 2 est recouverte d'un matériau à bouclettes souples 6, tel qu'un tricot ou un tissu gratté.

Les différentes matières premières utilisées sont, par exemple, les suivantes : pour la couche de matière plastique sous forme de mousse dure 2, du polyéthylène de 5 à 6 mm d'épaisseur ; pour la feuille métallique 3 de l'aluminium ; pour la couche de matière plastique sous forme de mousse souple 4, un mélange de polyuréthane et de polyester de 20 mm d'épaisseur ; et pour la feuille souple et lisse de protection, un polychlorure de vinyle. De préférence, la liaison entre la feuille métallique 3 et la couche de matière plastique sous forme de mousse dure 2 est assurée par une colle auto-adhésive pour des raisons que l'on comprendra mieux plus loin.

La feuille métallique 3, d'une épaisseur de l'ordre de 1 mm, est découpée en bandes longitudinales d'environ 25 mm de largeur, distinctes les unes des autres et sensiblement parallèles, c'est-à-dire qu'elles présentent une légère convergence vers l'une des extrémités du panneau qui est de forme générale trapézoïdale presque rectangulaire avec une base et une hauteur de l'ordre de 42 cm et 1 m, respectivement ; on trouve plusieurs, trois dans l'exemple, bandes longitudinales médianes 11, 12, 13 qui s'étendent sur une partie longitudinale médiane 14 du panneau, plusieurs, également trois dans l'exemple, bandes longitudinales latérales 15, 16, 17 qui s'étendent sur une partie longitudinale latérale 18 du panneau attenante à la partie longitudinale médiane 14 de ce dernier par une ligne 19 indiquée en traits mixtes, et enfin plusieurs, également trois par symétrie, bandes longitudinales latérales 22, 23, 24 qui s'étendent semblablement sur une partie longitudinale latérale 25 du panneau attenante à la partie longitudinale médiane 14 du panneau, de l'autre côté de celle-ci, par une ligne 26 indiquée aussi en traits mixtes. Les bandes métalliques longitudinales médianes 11, 12, 13 s'étendent pratiquement sur toute la longueur du panneau, mais les bandes métalliques longitudinales latérales 15, 16, 17 et 22, 23, 24 sont plus courtes à l'une de leurs extrémités qui correspond à l'extrémité distale de la gouttière. Les deux parties extrêmes 18A, 25A des deux parties longitudinales latérales 18, 25 du panneau, qui ne sont pas garnies de bandes métalliques, sont séparées de la partie extrême 14A de la partie longitudinale médiane 14 dudit panneau respectivement par deux fentes 28, 29, d'une longueur d'environ 22 cm, et elles sont repliées en deux sur elles-mêmes, au moment de la fabrication de l'article en usine comme indiqué en 25B sur la fig. 1.

Etant donné que la couche de matière plastique sous forme de mousse souple 4 présente un état de surface alvéolé limitant la surface de contact, pour qu'elle soit collée efficacement aux lames métalliques 3 et aux parties de la couche de matière plastique sous forme de mousse dure 2 non recouvertes par lesdites lames métalliques, on est conduit à pulvériser une colle au néoprène sur cette mousse souple 4, ainsi que sur les surfaces adjacentes des lames métalliques et de la couche de matière plastique sous forme de mousse dure 2, l'assemblage se faisant ainsi colle contre colle.

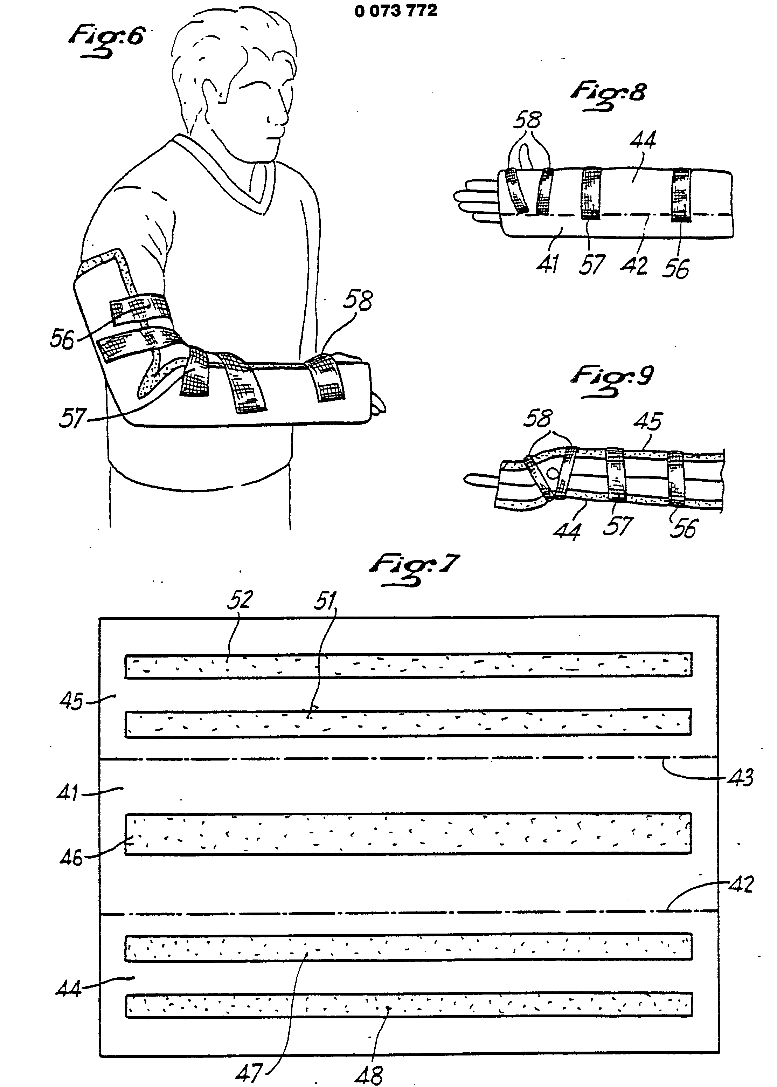

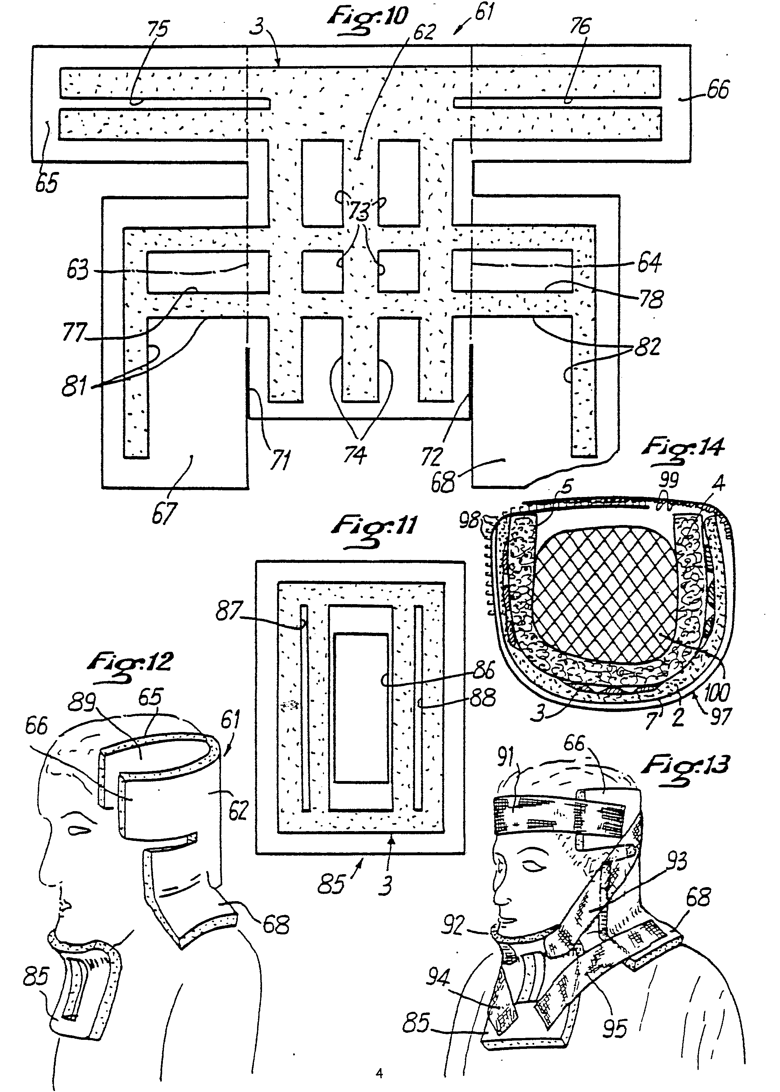

La mise en place de la gouttière sur un membre inférieur fracturé se fait facilement et permet de respecter une déformation éventuelle du membre ; on la glisse sous le membre, on relève les deux parties repliées 18A, 25A, à angle droit par rapport au plan du panneau, on relève semblablement, contre la plante du pied 38 du blessé, la partie extrême 14A de la partie longitudinale médiane du panneau, puis on relève et on modèle, contre les côtés du membre, les deux parties longitudinales latérales 18, 25 du panneau, en faisant passer les deux parties doublées 18A, 25A du côté de la face externe de la partie médiane relevée 14A du panneau de manière à fermer la gouttière en formant une sorte de boîte ouverte vers le haut qui sert d'appui pour le pied. Etant donné que les lames d'aluminium sont facilement déformables mais non élastiques, le moulage de la gouttière est fait en fonction de la déformation du foyer de fracture et ne présente pas de difficultés ; pour que la gouttière soit bien maintenue en place, il suffit de placer quelques sangles telles que 32, 33, 34, 35, 36 (Fig. 3 et 4) munie d'éléments souples et élastiques (du genre « Velcro » par exemple) formant crochets qui s'agrippent aux bouclettes du tissu gratté ou du tricot qui constitue la couche extérieure de la gouttière. On peut donc les accrocher à n'importe quel endroit de la face externe de la gouttière, ce qui facilite le respect de la déformation éventuelle.

La couche de matière plastique sous forme de mousse dure 2 amortit les déformations de la feuille d'aluminium qui sont une cause de rupture et répartit le modelage sur une surface plus grande ; cette propriété se trouve améliorée par la présence de la colle auto-adhésive dont il a été question plus haut, qui permet de refaire l'assemblage, même s'il arrive, à un moment donné, que les bandes d'aluminium et la mousse dure se trouvent disjointes sur une partie de leur surface.

La présence de la couche de matière plastique sous forme de mousse souple d'une épaisseur de 20 mm assure un rembourrage efficace, car sa densité est beaucoup plus faible que celle de la couche de matière plastique sous forme de mousse dure extérieure. La feuille intérieure souple et lisse de protection 5 en polychlorure de vinyle ou en tissu enduit à surface lisse, facilite le glissement de la gouttière au moment de sa mise en place et constitue un écran de protection de la mousse qui facilite l'entretien de la gouttière.

La gouttière est donc modelée à la main suivant la déformation du membre. Un grand avantage de cet article, par rapport à certaines attelles plates de structure apparemment analogue, réside surtout dans l'équilibre des forces d'action de la gouttière sur l'ensemble du membre inférieur enveloppé aux trois quarts, la résistance du volet transversal d'extrémité relevée, et la facilité d'adaptation des sangles de positionnement à tout emplacement désiré de la gouttière.

Sur la fig. 5, on peut voir un panneau de même structure générale, mais adapté à la confection d'une gouttière de maintien de la zone avant-bras, coude et partie inférieure du bras, la couche de matière plastique sous forme de mousse souple et la feuille intérieure de protection étant supposées transparentes pour qu'on puisse voir la structure interne du panneau, c'est-à-dire le découpage particulier de la feuille métallique. Le panneau, qui est rectangulaire, comporte une partie longitudinale médiane 41 attenante, par deux lignes droites théoriques 42, 43 dessinées en traits mixtes, à deux parties longitudinales latérales 44, 45, respectivement. La partie longitudinale médiane 41 est garnie, pratiquement sur toute sa longueur, d'une bande métallique centrale 46. Les parties longitudinales latérales 44, 45 sont garnies de deux bandes métalliques 47, 48 et 51, 52 qui s'étendent aussi pratiquement sur toute la longueur du panneau, mais qui présentent une interruption sur une partie intermédiaire de leur longueur, comme représenté. L'ensemble du panneau présente, sur ses deux côtés, deux échancrures 53, 54 au droit desdites interruptions des bandes métalliques, respectivement. Les emplacements des interruptions des bandes métalliques et des échancrures sont tels que l'ensemble de la gouttière se trouve divisé en deux parties d'inégales longueurs, à savoir : une partie courte destinée à être placée sur le bras (Voir Fig. 6) et une partie longue de maintien de l'avant-bras, qui sont reliées l'une à l'autre par la partie médiane étroite, à l'emplacement du coude.

La mise en place de cette gouttière se fait dans les mêmes conditions que celle de la gouttière de membre inférieur décrite plus haut, on la modèle suivant les déformations du bras blessé et on la maintient au moyen de sangles également 56, 57, 58. Les sangles sont croisées au niveau de la face antérieure du coude et permettent d'immobiliser le coude dans la position où il se trouve à l'accident. On n'est donc pas obligé d'étendre ou de fléchir le coude pour l'immobiliser. Cette gouttière est utilisable pour l'immobilisation du membre supérieur dans le cas de lésions osseuses ou articulaires intéressant le poignet, l'avant-bras, le coude et le tiers inférieur du bras.

Sur la fig. 7, on peut voir un panneau du même genre, mais un peu plus court, sans interruptions dans les bandes métalliques et sans échancrures latérales. Sur cette figure, on a conservé les mêmes chiffres de référence pour désigner les éléments correspondants à ceux du mode de réalisation de la fig. 5. Comme on peut le voir sur les fig. 8 et 9, ce type de gouttière est utilisable pour l'immobilisation de l'extrémité du membre supérieur dans le cas de lésions osseuses ou articulaires intéressant la main, le poignet et le tiers inférieur de l'avant-bras.

Sur les fig. 10 et 11, on a représenté respectivement les deux éléments d'une minerve, c'est-à-dire un appareil orthopédique destiné à maintenir la tête en rectitude. L'élément de la fig. 10 est l'élément principal désigné dans son ensemble par 61 ; il comporte une partie médiane 62 de forme rectangulaire dont les deux limites latérales sont définies par les deux lignes droites 63, 64 dessinées en traits mixtes, ainsi que deux ailettes latérales supérieures rectangulaires 65, 66 et deux ailettes latérales inférieures également rectangulaires 67, 68 toutes les quatre attenantes à la partie médiane 62 le long des deux lignes droites précitées 63, 64 correspondantes. Le bord supérieur des deux ailettes latérales supérieures 65, 66 est situé au même niveau que le bord supérieur de la partie médiane 62, tandis que, dans l'exemple, le bord inférieur des deux ailettes latérales inférieures se trouve un peu plus bas que le bord inférieur de la partie médiane. De plus, la partie inférieure des deux bords latéraux de la partie médiane 62 se trouve séparée de la partie adjacente des deux ailettes latérales inférieures 67, 68, par des fentes 71, 72, respectivement, pour faciliter le modelage de la minerve sur le patient. D'une manière générale, cette facilité est accrue par la présence, dans toutes les parties de la feuille métallique, d'ajours et/ou de fentes d'emplacements, de configurations et de dimensions adéquats, à savoir, par exemple : des ajours 73 dans la zone centrale de la partie médiane 62, des échancrures 74 dans la zone inférieure de cette même partie, des fentes 75, 76 respectivement dans les deux ailettes latérales supérieures 65, 66, des ajours 77, 78 et de très larges échancrures 81, 82, dans les deux ailettes latérales inférieures 67, 68, respectivement. De plus, dans toutes les parties de la feuille métallique, les bords de celle-ci se trouvent légèrement en retrait par rapport aux bords correspondants du panneau pour adoucir le contact de l'appareil sur la peau.

L'autre élément de la minerve, représenté sur la fig. 11, est une mentonnière constituée par un simple rectangle 85, toujours de la même structure que celle des panneaux dont il a été question jusqu'ici, avec un ajour central rectangulaire 86. Les bords de la feuille métallique 3 sont aussi en retrait par rapport aux bords correspondants de l'élément et deux fentes d'assouplissement 87, 88 sont prévues dans les deux grands côtés du rectangle.

Sur la fig. 12, on peut voir la manière d'utiliser la minerve : on exécute le modelage de l'élément principal 61 avant la pose en fonction de la position de la tête, l'autre élément 85 est appliqué et prend le relais de la main du sauveteur. On maintient le tout en place au moyen d'une bride frontale 91 qui s'accroche aux extrémités des deux ailettes latérales supérieures 65, 66 ; de chaque côté, une bride 92 ou 93 passe sur l'oreille obliquement et relie une ailette latérale supérieure 65 ou 66 à la partie marginale latérale correspondante de la mentonnière 85, tandis qu'une autre bride 94 ou 95 relie aussi une ailette latérale inférieure 67 ou 68 à l'autre partie marginale latérale de la mentonnière. En d'autres termes, cinq brides suffisent pour maintenir convenablement tout l'ensemble de l'appareil. La mentonnière permet de maintenir la tête dans l'inclinaison voulue ; l'ajour 86 (Fig. 11) évite toute compression sur la trachée et laisse libres les mouvements de déglutition. Comme pour les gouttières décrites plus haut, il n'y a aucun réglage à effectuer pour poser les brides, puisqu'on peut les accrocher en tout point des deux éléments de l'appareil. La réalisation de l'appareil en deux éléments permet sa mise en place par deux sauveteurs qui assurent, en même temps, le maintien de la tête et permettent une évacuation du blessé en toute sécurité.

Sur la fig. 14, on a représenté un autre mode de réalisation de gouttière fabriquée toujours à partir du même matériau de base mais dont la mousse de plastique dure 2 est recouverte, cette fois, non pas d'un matériau à bouclettes souples, tel que tricot ou tissu gratté comme dans les formes d'exécution décrites plus haut, mais d'une feuille extérieure souple et lisse 7, par exemple en tissu enduit. Dans ce cas, des sangles munies uniquement d'éléments d'accrochage ne pourraient évidemment pas s'agripper à la surface extérieure lisse de la gouttière ; on se servira alors, par exemple, de sangles telles que 97 dont une partie terminale est garnie d'éléments « Velcro » crochets 98 sur sa face externe, tandis que son autre partie terminale est garnie d'éléments « Velcro »

bouclettes 99 sur sa face interne, afin que ces deux parties terminales s'agrippent l'une à l'autre en maintenant solidement la gouttière qu'elles entourent. Une telle gouttière convient particulièrement pour le maintien d'un membre inférieur 100 et permet au sauveteur de travailler dans des conditions difficiles, par exemple dans la boue, la neige, ou sur terrain gelé.

Le nouveau matériau présenté, trouve, ainsi qu'on vient de l'exposer dans quelques exemples, une application directe dans la confection de dispositifs d'immobilisation pour des secours d'urgence, mais on peut l'utiliser avantageusement aussi pour confectionner des appareils de simple protection et de maintien de diverses parties du corps de l'homme et des animaux, par exemple la protection des jambes des chevaux pendant leur transport ou leur entraînement. On peut modifier les caractéristiques de sa structure en fonction des applications envisagées.

## Revendications

1. Matériau pour la confection d'articles de maintien de la tête ou de membres de l'homme ou des animaux, constitué par un panneau stratifié (1) formé par l'assemblage d'une couche extérieure de matière plastique sous forme de mousse (2), d'une âme intermédiaire en métal déformable et non élastique (3), et d'une couche intérieure de matière plastique sous forme de mousse souple (4), caractérisé en ce qu'il comporte en outre, une feuille intérieure extrême souple et lisse de protection (5), en ce que l'âme (3) est formée d'une feuille de métal et en ce que la couche extérieure (2) est en mousse dure.

2. Matériau selon la revendication 1, caractérisé en ce que la face distale de la couche de matière plastique sous forme de mousse dure (2) est recouverte d'un matériau à bouclettes souples (6), tel que tricot ou tissu gratté.

3. Matériau selon la revendicaiton 1, caractérisé en ce que la face distale de la couche de matière plastique sous forme de mousse dure (2) est recouverte d'une feuille extérieure souple et lisse (7).

4. Matériau selon la revendication 3, caractérisé en ce que la feuille extérieure souple et lisse est en tissu enduit.

5. Matériau selon la revendication 1, caractérisé en ce que la couche de matière plastique sous forme de mousse dure (2) est en polyéthylène.

6. Matériau selon la revendication 1, caractérisé en ce que la feuille de métal intermédiaire (3) est en aluminium.

7. Matériau selon la revendication 1, caractérisé en ce que la couche de matière plastique sous forme de mousse souple (4) est un mélange de polyuréthane et de polyester.

8. Matériau selon la revendication 1, caractérisé en ce que la feuille intérieure souple et lisse de protection (5) est en polychlorure de vinyle.

9. Matériau selon la revendication 1, caractérisé en ce que la liaison entre la feuille de métal (3) et la couche de matière plastique sous forme de mousse dure (2) est assurée par une colle auto-adhésive.

10. Matériau selon l'une quelconque des revendications 1 à 9, caractérisé en ce que la feuille de métal intermédiaire (3) est découpée suivant une forme prédéterminée qui correspond à la conformation de l'article à confectionner.

11. Matériau selon la revendication 10, destiné à la réalisation d'une gouttière, caractérisé en ce que la feuille de métal (3) est découpée en bandes longitudinales (11-13, 15-17, 22-24, 46, 47, 48, 51, 52) sensiblement parallèles et distinctes les unes des autres.

12. Matériau selon la revendication 11, pour la réalisation d'une gouttière destinée à protéger un membre inférieur, caractérisé en ce qu'il comporte une partie longitudinale médiane (14) garnie de bandes de feuilles de métal (11, 12, 13) sur toute sa longueur ainsi que deux parties longitudinales latérales (18, 25) attenantes à ladite partie longitudinale médiane (14) et de même longueur que cette dernière, garnies aussi de bandes de feuille de métal (15-17, 22-24) dont la longueur, à l'une de leurs extrémités, est plus courte que celle des bandes de feuille métallique de la partie longitudinale médiane (14), les parties non garnies de bandes de feuille métallique desdites bandes longitudinales latérales (18, 25) du matériau étant, chacune, séparée de la partie longitudinale médiane (14) par une fente (28, 29) pour pouvoir être appliquée contre l'extrémité adjacente de la partie longitudinale médiane du matériau relevée à angle droit contre la plante du pied appartenant au membre contre lequel sera roulé l'ensemble de la gouttière.

13. Matériau selon la revendication 11, pour la réalisation d'une gouttière destinée à être posée sur un avant-bras, le coude et la partie inférieure du bras, caractérisé en ce qu'il comporte une partie longitudinale médiane (41) garnie de bandes de feuille métallique (46) sur toute sa longueur ainsi que deux parties longitudinales latérales (44, 45) attenantes à ladite partie longitudinale médiane (41) et de même longueur que cette dernière, garnies de bandes de feuille métallique (47, 48 et 51, 52) qui présentent une interruption sur une partie intermédiaire de leur longueur, l'ensemble du panneau présentant, de chaque côté, une échancrure (53, 54) au droit de ladite interruption des bandes de feuille métallique.

14. Matériau selon la revendication 10, pour la réalisation d'une minerve de protection, caractérisé en ce qu'il comporte deux éléments, à savoir : un élément principal (61) et une mentonnière (85), l'élément principal comportant une partie médiane (62) de forme rectangulaire avec deux ailettes latérales supérieures (65, 66) destinées à être rabattues vers l'avant contre les tempes et deux ailettes latérales inférieures (67, 68) destinées à être modelées contre les côtés de la tête et sur le dessus des épaules, tandis que la mentonnière (85) est constituée d'un simple élément rectangulaire présentant un ajour central (86), la feuille métallique (3), dans toutes les parties des deux éléments de la minerve, étant ajourée et/ou fendue par places pour faciliter leur mise en forme.

15. Matériau selon l'une des revendications 2, 5-9, pour la réalisation d'une minerve de protection, caractérisé en ce que la feuille de métal (3) est découpée suivant une forme prédéterminée qui correspond à la conformation de la minerve, ladite minerve comportant, d'une part, deux éléments, à savoir : un élément principal (61) et une mentonnière (85), l'élément principal comportant une partie médiane (62) de forme rectangulaire avec deux ailettes latérales supérieures (65, 66) destinées à être rabattues vers l'avant contre les tempes et deux ailettes latérales inférieures (67, 68) destinées à être modelées contre les côtés de la tête et sur le dessus des épaules, tandis que la mentonnière (85) est constituée d'un simple élément rectangulaire présentant un ajour central (86), la feuille métallique (3), dans toutes les parties des deux éléments de la minerve, étant ajourée et/ou fendue par places pour faciliter leur mise en forme, et, d'autre part, des brides (91, 92, 93, 94, 95) munies d'éléments d'accrochage dans le matériau à bouclettes qui recouvre les deux éléments constitutifs de la minerve.

16. Matériau selon la revendication 15, caractérisé en ce qu'il est associé à cinq brides de fixation, à savoir : une bride frontale (91) qui s'accroche aux extrémités des deux ailettes latérales supérieures (65, 66), et, de chaque côté, une bride latérale (94 ou 95) qui s'accroche à l'ailette latérale inférieure correspondante (67 ou 68) et à la partie marginale correspondante de la mentonnière (85), ainsi qu'une autre bride latérale (92 ou 93) qui passe sur l'oreille du patient et s'accroche à l'ailette latérale supérieure correspondante (65 ou 66) et à la partie marginale correspondante de la mentonnière (85).

17. Matériau selon l'une des revendications 1, 2, 5-9, pour la réalisation d'une gouttière de protection d'un membre, caractérisé en ce que la feuille de métal (3) est découpée en bandes longitudinales (11-13, 15-17, 22-24, 46, 47, 48, 51, 52) sensiblement parallèles et distinctes les unes des autres, et en ce qu'il coopère avec des sangles (32, 33, 34, 35, 36, 38, 56, 57, 58) munies d'éléments d'accrochage dans le matériau à bouclettes souples (6) qui recouvre la face distale de la couche de matière plastique sous forme de mousse dure (2) dudit matériau.

18. Matériau selon l'une des revendications 1, 3-9, pour la réalisation d'une gouttière de protection d'un membre, caractérisé en ce que la feuille de métal (3) est découpée en bandes longitudinales (11-13, 15-17, 22-24, 46, 47, 48, 51, 52) sensiblement parallèles et distinctes les unes des autres, et en ce qu'il est associé à des sangles (97) munies de moyens (98, 99) d'accrochage mutuel de leurs parties terminales.

**Claims**

1. Material for the manufacture of articles for supporting the heads or members of persons or animals and constituted by a layered panel (1) formed by the assembly of an outer layer (2) of plastic material in the form of foam, an intermediary web (3) of deformable and inelastic metal, and an inner layer (4) of plastic material in the form of flexible foam, characterised in that it further comprises a flexible and smooth protective innermost sheet (5), in that the web (3) is formed by a metal sheet and in that the outer layer (2) is of hard foam.

2. Material according to Claim 1, characterised in that the distal face of the layer (2) of plastic material in the form of hard foam is covered with a flexible loop material (6), such as knitted or brushed fabric.

3. Material according to Claim 1, characterised in that the distal face of the layer (2) of plastic material in the form of hard foam is covered with a flexible and smooth external sheet (7).

4. Material according to Claim 3, characterised in that the flexible and smooth external sheet is of coated fabric.

5. Material according to Claim 1, characterised in that the layer (2) of plastic material in the form of hard foam is of polyethylene.

6. Material according to Claim 1, characterised in that the intermediary metal sheet (3) is of aluminium.

7. Material according to Claim 1, characterised in that the layer (4) of plastic material in the form of flexible foam is a mixture of polyurethane and polyester.

8. Material according to Claim 1, characterised in that the flexible and smooth internal protective sheet (5) is of vinyl polychloride.

9. Material according to Claim 1, characterised in that the binding between the metal sheet (3) and the layer (2) of plastic material in the form of hard foam is effected by a self-adhesive glue.

10. Material according to any one of Claims 1 to 9, characterised in that the intermediary metal sheet (3) is cut out to a predetermined form which corresponds to the shaping of the article to be manufactured.

11. Material according to Claim 10, for the manufacturing of a cradle, characterised in that the metal sheet (3) is cut into longitudinal strips (11-13, 15-17, 22-24, 46, 47, 48, 51, 52) which are substantially parallel and distinct from one another.

12. Material according to Claim 11, for the manufacturing of a cradle adapted to protect a lower member, characterised in that it comprises a median longitudinal part (14) provided with metal sheet strips (11, 12, 13) over its whole length and two lateral longitudinal parts (18, 25) contiguous with the said median longitudinal part (14) and of the same length as the latter, also provided with metal sheet strips (15-17, 22-24) the length of which, at one of their ends, is shorter than that of the metallic sheet strips of the median longitudinal part (14), the parts not provided with metallic sheet strips of the said lateral longitudinal strips (18, 25) of the material being each separated from the median longitudinal part (14) by a slot (28, 29) so that they can be applied against the adjacent end of the median longitudinal part of the material raised at right angles against the sole of the foot pertaining to the member against which the cradle assembly will be rolled.

13. Material according to Claim 11, for the manufacturing of a cradle adapted to be placed upon a forearm, the elbow and the lower part of the arm, characterised in that it comprises a median longitudinal part (41) provided with metallic sheet strips (46) over its entire length and two lateral longitudinal parts (44, 45) contiguous with the said median longitudinal part (41) and of the same length as the latter, provided with metallic sheet strips (47, 48 and 51, 52) which have an interruption on an intermediate part of their length, the panel assembly having on each side a cut-out part (53, 54) in line with the said interruption of the metallic sheet strips.

14. Material according to Claim 10, for the manufacturing of a protective neck brace, characterised in that it comprises two elements, namely : — a main element (61) and a chin-piece (85), the main element comprising a median part (62) of rectangular form with two upper lateral wings (65, 66) intended to be turned forward against the temples and two lower lateral wings (67, 68) intended to be modelled against the sides of the head and over the top of the shoulders, while the chin-piece (85) is constituted by a simple rectangular element having a central orifice (86), the metallic sheet (3), in all the parts of the two elements of the neck brace, being orificed and/or slotted in places to facilitate their shaping.

15. Material according to one of Claims 2, 5-9, for the manufacturing of a protective neck brace, characterised in that the metal sheet (3) is cut out in a predetermined form corresponding to the conformation of the neck brace, the said neck brace comprising on the one hand two elements, namely : a main element (61) and a chin-piece (85), the main element comprising a median part (62) of rectangular form with two upper lateral wings (65, 66) intended to be turned forward against the temples and two lower lateral wings (67, 68) intended to be modelled against the sides of the head and over the top of the shoulders, while the chin-piece (85) is constituted by a simple rectangular element having a central orifice (86), the metallic sheet (3), in all parts of the two elements of the neck brace, being orificed and/or slotted in places to facilitate their shaping, and furthermore straps (91, 92, 93, 94, 95) provided with elements for hooking into the looped material covering the two constituent elements of the neck brace.

16. Material according to Claim 15, characterised in that it is associated with five fastening straps, namely : a forehead strap (91) which is hooked to the ends of the two upper lateral wings (65, 66) and on each side a lateral strap (94 or 95) which hooks itself to the corresponding lower lateral wing (67 or 68) and to the corresponding marginal part of the chin-piece (85), and another lateral strap (92 or 93) which passes over the ear of the patient and is hooked to the corresponding upper lateral wing (65 or 66) and to the corresponding marginal part of the chin-piece (85).

17. Material according to one of claims 1, 2, 5-9, for the manufacturing of a cradle for the protection of a member, characterised in that the metal sheet (3) is cut out into longitudinal strips (11-13, 15-17, 22-24, 46, 47, 48, 51, 52) which are substantially parallel and distinct from one another, and in that it cooperates with straps (32, 33, 34, 35, 36, 38, .56, 57, 58) provided with elements for hooking into the flexible looped material (6) covering the distal face of the layer (2) of plastic material in the form of hard foam of the said material.

18. Material according to one of claims 1, 3-9, for the manufacturing of a cradle for the protection of a member, characterised in that the metal sheet (3) is cut into longitudinal strips (11-13, 15-17, 22-24, 46, 47, 48, 51, 52) which are substantially parallel and distinct from one another, and in that it is associated with straps (97) provided with means (98, 99) for mutual hook attachment of their terminal parts.

**Patentansprüche**

1. Material zur Herstellung von Teilen zum Stützen des Kopfes oder der Gliedmassen von Menschen oder Tieren, bestehend aus einer geschichteten Bahn (1), die durch den Zusammenbau aus einer äusseren Kunststoffschicht aus Schaum (2), einer deformierbaren und nicht elastischen Zwischenmetallseele (3) und aus einer inneren Kunststoffschicht aus biegsamen Schaum (4) gebildet wird, dadurch gekennzeichnet, dass es ausserdem eine weiche und glatte am meisten innere Schutzschicht (5) enthält und dass die Seele (3) von einer Metallfolie gebildet wird und dass die Aussenschicht (2) aus Hartschaum ist.

2. Material nach Anspruch 1, dadurch gekennzeichnet, dass die körperferne Fläche der Kunststoffschicht aus Hartschaum (2) mit einem Material mit weichen Schlaufen (6) wie z. B. Trikot oder aufgerauhtes Gewebe, versehen ist.

3. Material nach Anspruch 1, dadurch gekennzeichnet, dass die körperferne Fläche der Kunststoffschicht aus Hartschaum (2) mit einer weichen, glatten Aussenschicht (7) versehen ist.

4. Material nach Anspruch 3, dadurch gekennzeichnet, dass die weiche und glatte Aussenschicht ein glattes Gewebe ist.

5. Material nach Anspruch 1, dadurch gekennzeichnet, dass die Kunststoffschicht aus Hartschaum (2) ein Polyäthylen ist.

6. Material nach Anspruch 1, dadurch gekennzeichnet, dass die Zwischenmetallfolie (3) aus Aluminium besteht.

7. Material nach Anspruch 1, dadurch gekennzeichnet, dass die Kunststoffschicht aus weichem Schaum (4) eine Mischung aus Polyurethan und Polyester aufweist.

8. Material nach Anspruch 1, dadurch gekennzeichnet, dass die innere weiche und glatte Schutzschicht (5) aus Polyvinylchlorid besteht.

9. Material nach Anspruch 1, dadurch gekennzeichnet, dass die Verbindung zwischen der Metallfolie (3) und der Kunststoffschicht aus Hartschaum (2) durch einen selbstklebenden Klebstoff erfolgt.

10. Material nach einem der vorhergehenden Ansprüche 1 bis 9, dadurch gekennzeichnet, dass die Zwischenmetallfolie (3) auf eine vorgegebene Form zugeschnitten ist, die der Ausbildung des herzustellenden Teils entspricht.

11. Material nach Anspruch 10, bestimmt zur Herstellung einer Schutzschiene, dadurch gekennzeichnet, dass die Metallfolie (3) in Längsstreifen (11 bis 13, 15 bis 17, 22 bis 24, 46, 47, 48, 51, 52) geschnitten ist, die im wesentlichen parallel und getrennt voneinander angeordnet sind.

12. Material nach Anspruch 11, zur Herstellung einer Schiene bestimmt zum Zwecke des Schutzes eines unteren Gliedmasses, dadurch gekennzeichnet, dass es ein mittleres Längsteil 14 aufweist, das mit Metallbändern (11, 12, 13) über seine gesamte Länge versehen ist, sowie mit seitlichen Längsteilen (18, 25), welche an das mittlere Längsteil (14) angrenzen und eine gleiche Länge wie dieses aufweisen, wobei letztere ebenfalls mit Metallstreifen (15 bis 17, 22 bis 24) versehen sind, deren Länge an einem Ende kürzer ist als diejenige der Metallbänder des mittleren Längsteils (14), wobei die nicht mit Metallbändern versehenen Teile der seitlichen Längsteile (18, 25) des Materials jeweils von dem mittleren Längsteil (14) durch einen Schlitz (28, 29) getrennt sind um gegen das benachbarte Ende des mittleren Längsteils des umgebogenen Materials in einem rechten Winkel gegen die Fussohle angelegt zu werden, die zu demjenigen Gliedmass gehört, um das die Schiene herumgerollt wird.

13. Material nach Anspruch 11, zur Herstellung einer Schiene bestimmt zur Anbringung auf einem Vorderarm, Ellbogen und Oberarm, dadurch gekennzeichnet, dass es ein mittleres Längsteil (41) aufweist, das mit Metallbändern (46) über seine gesamte Länge versehen ist sowie zwei seitliche Längsteile (44, 45) aufweist, die an das mittlere Längsteil (41) angrenzen und gleiche Länge wie dieses aufweisen und mit Metallbändern (47, 48, 51, 52) versehen sind, die eine Unterbrechung an einem Mittelteil ihrer Längsausdehnung aufweisen, wobei die Gesamtheit der Bahn an jeder Seite eine Aussparung (53, 54) gegenüber der Unterbrechung der Metallbänder aufweist.

14. Material gemäss Anspruch 10 zur Herstellung einer Kopfstütze, dadurch gekennzeichnet, dass es zwei Teile aufweist, nämlich : ein Hauptteil (61) und eine Kinnstütze (85), wobei das Hauptteil ein mittleres rechtwinkliges Teil (62) mit zwei oberen seitlichen Flügeln (65, 66) aufweist, welche nach vorne in Richtung der Schläfen umklappbar sind, sowie zwei untere seitliche Flügel (67, 68) aufweist, die dazu bestimmt sind, an den Kopfseiten und auf der Oberseite der Schlutern anzuliegen, während die Kinnstütze (85) aus einem einfachen rechtwinkligen Teil besteht mit einer zentralen Öffnung (86), wobei die Metallfolie (3) an allen Stellen der beiden Teile der Kopfstütze mit Öffnungen und/oder Schlitzen versehen ist, um ihr Anlegen zu erleichtern.

15. Material nach einer der Ansprüche 2, 5-9 zur Herstellung einer Kopfstütze, dadurch gekennzeichnet, dass die Metallfolie (3) gemäss einer vorgegebenen Form zugeschnitten ist, die der herzustellenden Kopfstütze entspricht und wobei die Kopfstütze einerseits zwei Teile aufweist, nämlich : ein Hauptteil (61) und eine Kinnstütze (85), wobei das Hauptteil ein rechtwinkliges mittleres Teil (62) aufweist, mit zwei oberen seitlichen Flügeln (65, 66), die dazu bestimmt sind, nach vorne und gegen die Schläfen umgeklappt zu werden und zwei untere seitliche Flügel (67, 68), die dazu bestimmt sind, gegen die Kopfseiten und die Oberflächen der Schultern angelegt zu werden, während die Kinnstütze (85) aus einem einfachen rechtwinkligen Teil besteht mit einer zentralen Öffnung (86), während die Metallfolie (3) an allen Stellen der beiden Teile der Kopfstütze mit Öffnungen und/oder Schlitzen versehen ist um ihr Anlegen zu erleichtern sowie andererseits Gurte (91, 92, 93, 94, 95) aufweist, die mit Halteelementen für das mit Schlingen versehene Material versehen ist, welche die beiden Teile der Kopfstütze bedecken.

16. Material nach Anspruch 15, dadurch gekennzeichnet, dass sie fünf Haltegurte aufweist, nämlich ; einen Stirngut (91), der an den beiden Enden der oberen seitlichen Flügel (65, 66) befestigt ist und auf jeder Seite einen Seitengurt (94 oder 95), der sowohl an dem entsprechenden unteren seitlichen Flügel (67 oder 68) und an dem entsprechenden Randstück der Kinnstütze (85) befestigt ist, sowie einen weiteren seitlichen Gurt (92 oder 93), der über das Ohr des Patienten verläuft und sowohl am oberen seitlichen entsprechenden Flügel (65 oder 66) und an dem entsprechenden Randstück der Kinnstütze (85) befestigt ist.

17. Material nach einer der Ansprüche 1, 2, 5-9 zur Herstellung einer Schutzschiene für ein Gliedmass, dadurch gekennzeichnet, dass die Metallfolie (3) in Längsbänder (11 bis 13, 15 bis 17, 22 bis 24, 46, 47, 48, 51, 52) unterteilt ist, die im wesentlichen parallel zueinander aber getrennt voneinander angeordnet sind und dass sie Gurte (32, 33, 34, 35, 36, 38, 56, 57, 58) aufweist, die mit Halteelementen für das mit Schlingen versehene weiche Material (6) versehen ist, welches die körperferne Fläche der Kunststoffschicht aus Hartschaum (2) des Materials bedeckt. .

18. Material nach einer der Ansprüche 1, 3-9 zur Herstellung einer Schutzschiene, dadurch gekennzeichnet, dass die Metallfolie (3) in im wesentlichen parallele aber getrennt voneinander angeordnete Längsbänder (11 bis 13, 15 bis 17, 22 bis 24, 46, 47, 48, 51, 52) unterteilt ist und dass sie mit Gurten (97) versehen ist, die Halteelemente (98, 99) aufweisen, welche ihre Endteile miteinander verbinden.

Fig.1

28  14A  29

25A

18A

25B

1

19

26

II                II

18                25

15                22

16                23

17                24

11  14  12  13

Fig.2

15  16  5  17  3  4  11  12  13  1  24  23  22

I                I

2  6

0 073 772

Fig.3

Fig.4

Fig.5

*Fig:6*

*Fig:8*

58

44

41  57  42  56

58

56

57

*Fig:9*

58

45

44  57  56

*Fig:7*

52  51

45

43

41

46

42

44

47  48

3

Fig:10

Fig:14

Fig:11

Fig:12

Fig:13